(19) **Europäisches Patentamt**
**European Patent Office**
**Office européen des brevets**

(11) **EP 1 389 466 B1**

(12) **EUROPEAN PATENT SPECIFICATION**

(45) Date of publication and mention
of the grant of the patent:
**21.02.2007 Bulletin 2007/08**

(51) Int Cl.:
***A61K 9/50*** *(2006.01)* ***A61P 3/10*** *(2006.01)*

(21) Application number: **02722816.2**

(86) International application number:
**PCT/JP2002/004205**

(22) Date of filing: **26.04.2002**

(87) International publication number:
**WO 2002/087603 (07.11.2002 Gazette 2002/45)**

(54) **A dispersion on the basis of beta-glucan containing superfine particles, a corresponding process of manufacturing and the use of said dispersion**

Eine Dispersion aus Beta-Glukan enthaltenden hochfeinen Partikel, ein entsprechender Herstellungsprozess und die Verwendung der Dispersion

Une dispersion sur la base des particules superfines contenant beta glucane, la procédure correspondante de la fabrication et l'emploi de cettes dispersion

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.04.2001 JP 2001132513**

(43) Date of publication of application:
**18.02.2004 Bulletin 2004/08**

(73) Proprietor: **Ajinomoto Co., Inc.**
**Tokyo 104-8315 (JP)**

(72) Inventors:
• **SUGA, Tetsuya**
**c/o AJINOMOTO CO., INC.**
**Chuo-ku, Tokyo 104-8315 (JP)**
• **OGASAWARA, Yoshiaki**
**c/o AJINOMOTO CO., INC.**
**Chuo-ku, Tokyo 104-8315 (JP)**
• **KANEKO, Yutaro**
**c/o AJINOMOTO CO., INC.**
**Chuo-ku, Tokyo 104-8315 (JP)**
• **KAJIURA, Masatoshi**
**Tech.&Engine.Cen.,Ajinomoto,Inc**
**Kawasaki-shi, Kanagawa 210-0801 (JP)**
• **SUGA,Y.**
**Pharmaceutical Res.Lab.Ajinomoto Co., Inc.**
**Kawasaki-shi, Kanagawa 210-0801 (JP)**

(74) Representative: **Nicholls, Kathryn Margaret et al**
**Mewburn Ellis LLP**
**York House**
**23 Kingsway**
**London WC2B 6HP (GB)**

(56) References cited:
**WO-A-02/12348** **WO-A1-00/03724**
**WO-A1-87/01037** **WO-A1-96/28476**
**WO-A1-97/01330** **DE-A- 3 019 614**
**DE-A- 19 944 695** **GB-A- 1 356 449**
**JP-A- 2001 112 436** **US-A- 5 849 720**
**US-A1- 2001 000 229**

• **ARTURSSON P EDMAN P ERICSSON J L-E: "Macrophage stimulation with some structurally related polysaccharides" 1987, SCANDINAVIAN JOURNAL OF IMMUNOLOGY, BLACKWELL SCIENCE PUBL., OXFORD, GB, PAGE(S) 245-254 , XP002955616 ISSN: 0300-9475 * abstract * * page 246, right-hand column, line 27 - page 247, left-hand column, line 12 * * page 251, right-hand column, line 23 - page 252, right-hand column, line 25 ***
• **ARTURSSON P. ET AL.: 'Macrophage stimulation with some structurally related polysaccharides' SCAND. J. IMMUNOL. vol. 25, no. 3, 1987, pages 245 - 254, XP002955616**

**EP 1 389 466 B1**

**Description**

Technical Field

The present invention relates to novel superfine particles of a mushroom-derived component such as novel superfine particles of a mushroom extract etc. (preferably, superfine particles of a component obtained by extraction from a mushroom with water) or novel superfine particles of β-glucan, a composition comprising the novel superfine particles (dispersion of the superfine particles, etc.), an immune activator and/or an immune regulator comprising the superfine particles or the composition as an active ingredient (immune activator/immune regulator), a pharmaceutical composition (particularly, pharmaceutical preparations for diseases starting (occurring) due to abnormalities in immune functions, such as an antitumor agent, an anti-infective agent, an antiviral agent, an anti-autoimmune disease agent, an anti-diabetes agent and an anti-allergy agent, as well as pharmaceutical preparations for digestive organ diseases (therapeutic agents for irritable bowel syndrome (IBS), inflammatory bowel disease (IBD), constipation, diarrhea and the like)), food and drink (health foods, functional foods etc.), and a process for producing the superfine particles usable as an active ingredient for these diseases or a composition comprising the superfine particles.

The immune activator/immune regulator of the present invention is used in various forms such as a pharmaceutical preparation (pharmaceutical composition), food and drink (health foods, functional foods etc.) etc., and is useful for treatment, amelioration, and prevention from progression, of diseases particularly by activating or regulating immune functions, for prophylaxis of other diseases occurring due to abnormalities in immune functions in (for) patients and the like, and for prophylaxis of various diseases accompanying abnormalities in immune functions for healthy persons by activating or regulating immune functions, and for amelioration of light diseases by improving immune functions therefor, and the like.

The present invention also encompasses a method of activating immunity or a method of regulating immunity, which is useful for treatment (medical treatment), amelioration, prevention from progression and prophylaxis, etc. of tumors, infections, viral infections (diseases), autoimmune diseases, diabetes, allergic diseases, and digestive organ diseases (irritable bowel syndrome (IBS), inflammatory bowel disease (IBD), constipation, diarrhea and the like) and a use of the novel superfine particles as an active ingredient for the immune (immunity) activator, the immune (immunity) regulator, and various chemicals (agents), and for production of foods, drinks, etc. and further pharmaceutical preparations, and the like.

Background Art

Mushrooms or components thereof contain various pharmaceutically efficacious components, and thus health foods containing processed powder of a certain mushroom or a hot-water extract thereof are known. In the conventionally known products, however, their various components are not sufficiently utilized.

Accordingly, it is expected that various components of mushrooms or similar materials are utilized effectively much more in the form of pharmaceutical preparations, health foods or functional foods in comparison to the conventional products, in order to maintain and improve the health of animals, particularly humans in daily life, or used in pharmaceutical preparations to treat or ameliorate diseases.

US20010000229 A1 discloses a pharmaceutical composition as well as food or animal food which comprise *Grifola* powder or extracts obtained from fresh or dried *Grifola* (*Grifola frondosa, Grifola albicans, Grifola gigantea*) by using water or water/organic solvent as extracts. The extract contains a polysaccharide (e.g. beta glucan) or a complex of a polysaccharide and a protein. The *Grifola* extract or a purified fraction thereof is mixed with a water soluble or hot water soluble excipient (dextrin, cyclodextrin or lactose). Pharmaceutical nutraceutical or nutritional compositions comprising *Grifola* are used for the manufacture of a medicament or of food for chemoprevention of cancer.

In Scand J immunol. 1987. 25. 245-254 microparticles of 1,3-beta-glucan are described, which are used in a comparison of the stimulation of interleukin 1 and macrophage-mediated tumour cell killing by this and other polysaccharides in microparticulate form.

In DE 3019614 hydrolysed beta-(1,3) (1,6)-glucan - isolated from a fungus belonging to the *corticiaceae* - is shown to be incorporated in pharmaceutical compositions for parenteral administration and to have activity against specific types of cancer and tumours.

DE 19944695 discloses compositions and a fermentation process for isolating/extracting beta glucan from fungi (Agaricus, Maitake, Shiitake, Matsutake, Shimejitake) with an anti-tumour activity in cases of an oral intake.

In US 5849720 yeast-derived glucan particles were solubilised, and the soluble glucan used for the manufacture of a pharmaceutical composition for parenteral, topical, oral or intranasal administration to treat immunocompromised patients.

In GB 1356449 a process for the extraction of water soluble components of the hyphae of edible fungi (Shiitake, *Lentinus edodes, Basidiomycetes)* was described which included the formation of an aqueous suspension of fine particles, filtering the suspension to remove undesirable matter therefrom and thus providing an aqueous solution of an extract of the edible fungus. The composition is used for the manufacture of a medicament to treat tonsillitis, high blood pressure and cancer.

Earlier application WO0212348 (filed 31 July 2001) shows glucan particles (size range of 0.1-25 micrometers, preferably 0.5-15 micrometers, most preferably 2-10 micrometers, source: yeast) for the preparation of formulations to treat cardio-vascular diseases, to improve autoimmune conditions, arthritis, lupus, allergic asthma or multiple sclerosis.

Object of Invention/Problem before Invention

The object of the present invention is to provide food and drink (health foods, functional foods etc.) or a pharmaceutical preparation (pharmaceutical composition), which can be prepared by easy preparative means and effectively utilize various components particularly pharmaceutically efficacious components in mushrooms or similar materials.

Disclosure of Invention

To solve the problem described above, the present inventors made extensive study on utilization of various components in conventional products, and as a result, they found that the various components were not sufficiently absorbed into animal bodies, and were thus not so utilized as to be expected in the bodies. As a result of further investigation, the inventors found that when components derived from a mushroom, particularly an extract of a mushroom with water, preferably an extract thereof with hot water, were further finely pulverized to prepare superfine particles and dispersed, for example in water such that an average particle diameter of the superfine particles was 10 $\mu$m or less, more preferably 1 $\mu$m or less, still more preferably 0.01 to 1 $\mu$m in a micellar state, incorporation thereof through mucosa was significantly improved, and as a result, immune functions could be activated or regulated. Further, the inventors found that the same activity and action were present in superfine particles of $\beta$-glucan (including $\beta$-glucan derived from a mushroom and $\beta$-glucan not derived from a mushroom).

On the basis of the findings described above, the present invention was arrived at. Particularly, mucosal immunity is stimulated and activated by incorporation through mucosa (particularly the small intestine) into the body (leading to activation of systemic immunity), and as a result, an antitumor effect or a therapeutic treating and/or ameliorating effect on infectious diseases with viruses, such as AIDS, and bacteria or the like, can be expected. Accordingly, the superfine particles can be used as an immune activator/immune regulator (which is an immune activator and/or an immune regulator), and use thereof in the form of pharmaceutical composition(s) or food(s) and drink(s) (which is/are foods(s) and/or drink(s)) (health foods etc.) can be expected.

That is, one aspect of the present invention lies in superfine particles characterized in that a component selected from a component derived from a mushroom and $\beta$-glucan is converted into superfine particles, for example superfine particles characterized in that a component derived from a mushroom or $\beta$-glucan is converted into superfine particles. As the prior art relating to the present invention, there is $\beta$-glucan encapsulated in liposomes (see International Patent Publication WO 01/85141), but in the present invention, a component selected from a component derived from a mushroom and $\beta$-glucan may be in the form of superfine particles, and thus the present invention is evidently different from this prior art in that the $\beta$-glucan preparation can be produced by a simpler process without requiring procedures required in the prior art, such as the procedure of encapsulating $\beta$-glucan into liposomes and the procedure of separating, from the liposomes, $\beta$-glucan not encapsulated in the liposomes by gel permeation column chromatography etc. Accordingly, the present invention does not encompass the form of liposome characterized in that the active ingredient $\beta$-glucan is encapsulated therein.

In the present invention, the "component derived from a mushroom" refers collectively to component(s) produced by, in and/or from mushroom(s).

The component derived from a mushroom is not particularly limited insofar as it is a component contained in, or produced by and/or from, a mushroom. For example, the component derived from a mushroom may be an extract containing plural kinds of components contained in a mushroom and/or mushrooms, such as a mushroom extract or an extract of a mushroom with water. Further, the component derived from a mushroom may be a culture product produced in a culture solution by methods of culturing mycelia as described in Japanese Patent Publications JP-B-42-12000, JP-B-46-37873, JP-A-10-287584 etc. The component derived from a mushroom may be a single component such as $\beta$-glucan. It may also be a material containing $\beta$-glucan.

On one hand, $\beta$-glucan used in the present invention may be a component derived from a mushroom as described above, or may be $\beta$-glucan not contained in a component derived from a mushroom.

The $\beta$-glucan not contained in the component derived from a mushroom includes $\beta$-glucan as a component derived from e.g. yeast(s) (beer yeast etc.), a component derived from fungi, a component derived from bacteria, a component derived from plant(s) etc.

The extract of a mushroom is preferably an extract thereof with water (a water extract of a mushroom) (including extracts with water, hot water, a water-containing solution, etc.) in order to obtain a larger amount of the active (effective) ingredient in the present invention. The extract of a mushroom with water (the water extract of a mushroom) may be component(s) extracted from a mushroom with water or a material containing the component (s), and the extract includes, for example, a filtrate obtained by filtrating a water extract of a mushroom through a filter paper (the type of filter paper used can be selected as necessary without particular limitation) or the like, components contained therein (in the from of solid(s) or an aqueous solution, etc.), and a dispersion containing, in the filtrate, a part of the water-extracted component (s) in the form of dispersed fine particles (e.g. precipitates of aggregates each having a particle diameter of 100 $\mu$m or more, etc.). Thereafter, such a water extract of a mushroom can be subjected to the step of superfine pulverization in the present invention for superfine particles preparation. The aqueous solution containing these components is referred to as an aqueous solution containing a water extract of a mushroom, but it is not always necessary for these components to be completely dissolved in the aqueous solution.

On one hand, $\beta$-glucan used as the other starting material subjected to the step of superfine pulverization may be a $\beta$-glucan extract (extracted $\beta$-glucan component(s)) as is the case with the mushroom extract described above. Extraction of $\beta$-glucan can be carried out by extraction methods known in the art, for example a method of extraction with water or hot water (see Biol. Pharm. Bull., 23(7), 866, 2000), a method of treatment with an enzyme (see Japanese Patent Kokai Publications JP-A-5-268905, JP-A-10-287584 etc.) etc. The aqueous solution containing $\beta$-glucan extract is referred to as an aqueous $\beta$-glucan extract-containing solution, but it is not always necessary for the $\beta$-glucan extract to be completely dissolved in the aqueous solution.

In the case of superfine pulverization of a component selected from a component derived from a mushroom and $\beta$-glucan, the component preferably forms an aggregate in an aqueous solution. More preferably, the aggregate has a particle diameter of at least 50 $\mu$m (50 $\mu$m or more).

The superfine particles in the present invention have an average particle diameter of preferably 10 $\mu$m or less, more preferably 1 $\mu$m or less, still more preferably 0.01 to 1 $\mu$m or so as determined after being dispersed in water. This average particle diameter can be determined easily with a particle size distribution meter.

Some examples of such superfine particles are as follows:

(1) Superfine particles in a state treated or dispersed with a dispersant preferably in an aqueous solution, which are obtained from a component selected from a component derived from a mushroom and $\beta$-glucan.

(2) Superfine particles comprising particles having an average particle diameter of 10 $\mu$m or less, which can be obtained upon mixing a dispersant with an aqueous solution containing a component selected from a component derived from a mushroom and $\beta$-glucan.

(3) The superfine particles described in the above-mentioned (1) or (2), which comprise particles having an average particle diameter of 1 $\mu$m or less obtainable or obtained by a finely pulverizing treatment.

(4) The superfine particles described in the above-mentioned (3), wherein the average particle diameter is 0.01 to 1 $\mu$m.

(5) The superfine particles described in any of the above-mentioned (1) to (4), wherein the aqueous solution containing a component selected from a component derived from a mushroom and $\beta$-glucan is an aqueous mushroom extract-containing solution obtained by filtration, through a filter paper etc., of an extract (solution) of a mushroom with water or hot water.

(6) The superfine particles described in the above-mentioned (5), wherein the aqueous mushroom extract-containing solution is an aqueous solution containing aggregates obtained by filtering an extract (solution) of a mushroom with water or hot water through a paper filter etc. and then concentrating and/or cooling the filtrate.

(7) The superfine particles described in any of the above-mentioned (1) to (6), wherein the aqueous solution containing a component selected from a component derived from a mushroom and $\beta$-glucan is an aqueous solution of $\beta$-glucan or an aqueous solution containing $\beta$-glucan.

The $\beta$-glucan may be a component contained, or may not be a component contained in components derived from a mushroom.

The aqueous solution containing a component selected from a component derived from a mushroom and $\beta$-glucan is not particularly limited insofar as it is an aqueous solution containing a component selected from a component derived from a mushroom and $\beta$-glucan, and it is not necessary that the component be dissolved completely in the aqueous solution. For example, it may be an aqueous mushroom extract-containing solution or an aqueous solution containing a water extract of a mushroom or may be a culture solution containing culture products of mushroom mycelia. The aqueous solution is not particularly limited to a solution containing only water other than a component derived from a mushroom, but thus the solution may contain another component in addition to water and the component selected from a component derived from a mushroom and $\beta$-glucan. The aqueous solution containing $\beta$-glucan (component) is not particularly limited either, and may be an aqueous solution containing $\beta$-glucan, and it is not necessary for $\beta$-glucan to be completely dissolved in the aqueous solution, and an aqueous $\beta$-glucan-containing solution can be used. As described above, this β-glucan may be a component derived from a mushroom or may not be the one contained in components derived from a mushroom. Accordingly, the β-glucan extract component described above can be used therefor.

As to a quantity consumed of dispersant, a dispersant can be mixed with the aqueous solution containing the component(s) (or total components) selected from a component derived from a mushroom and β-glucan such that the weight ratio of the dispersant to the whole sugar (1) (total amount of sugar) contained in the aqueous solution containing the component (s) selected from a component derived from a mushroom and β-glucan is preferably at most 100 (100 or less), more preferably 10 or less, still more preferably 0.05 to 5 or so.

The content of a component selected from a component derived from a mushroom and β-glucan in the aqueous solution is not particularly limited, but it is preferable for solubility that the content of the component(s) (or total components) selected from a component derived from a mushroom and β-glucan in the aqueous solution can be determined such that the concentration of the whole sugar is at most 50 mg/ml (not higher than 50 mg/ml), more preferably in the range of 0.5 to 50 mg/ml or so.

The type etc. of the dispersant is not particularly limited, and examples of the dispersant include surfactants, polymers, sugars, sugar alcohols, glycerides, acids, bases, salts, etc. Among these, an emulsifier as a typical example of the surfactants is preferable, and lecithin can be used more preferably.

The superfine particles of the present invention can be obtained and used in the state of micelles with an emulsifier.

Although the method of finely pulverizing treatment is not particularly limited, a wet milling process with a high-pressure emulsifier (homogenizer), a media mill, supersonic waves (sonicator) etc. is preferable. For example, the high-pressure emulsifier can be used to prepare superfine particles of 1 $\mu$m or less described above. The emulsifying pressure used is preferably at least 300 kgf/cm$^2$, more preferably at least 500 kgf/cm$^2$, still more preferably at least 800 kgf/cm$^2$, and the emulsifying pressure can be reduced by increasing the number of times the treatment was repeated.

The superfine particles can be obtained by filtering preferably an extract (solution) of a water or hot-water mushroom or an extract solution of β-glucan through a filter paper etc. to give an aqueous solution containing a mushroom extract or β-glucan extract, mixing a dispersant therewith preferably under stirring. The superfine particles containing particles having an average particle diameter of 10 $\mu$m or less can be obtained in this manner.

In this case, it is possible to use, for example, not only an aqueous solution containing aggregates obtained by filtering a water or hot-water extract of a mushroom as the aqueous mushroom extract-containing solution (the aqueous solution containing a mushroom extract) through e.g. a filter paper etc. and then concentrating and/or cooling the filtrate, but also the aqueous filtrate which will, upon being concentrated and/or cooled, give the aggregates. The β-glucan can also be used by preparing the same aqueous solution as described above by the same treatment as above.

For use as an immune activator or an immune regulator, the superfine particles can be used in the form of an aqueous solution or dispersion in which the active ingredient is finely pulverized for example (mushroom extract treated with a dispersant as described above, etc.), desirably in the form of micelles. Among these, the mushroom extract or β-glucan treated with a dispersant can be conveniently used. Thus, the superfine particles in such various forms also fall under the scope of the superfine particles of the present invention.

The extract with hot water, for example the extract of a mushroom with hot water, is preferable from the viewpoint of efficient extraction of the active (effective) ingredient with hot water from a mushroom after milling.

The superfine particles can be absorbed or incorporated (ingested) through mucosa in small intestines of animals particularly humans, thus exhibiting an immune activating effect or an immune regulating effect.

Another aspect of the present invention lies in an immune activator and/or an immune regulator characterized by comprising any superfine particles described above (immune activator/immune regulator).

A still other aspect of the present invention lies in a pharmaceutical composition characterized by comprising any superfine particles described above (agent (medicine); pharmaceutical preparation). The pharmaceutical composition may contain a pharmaceutically acceptable carrier, excipient (bulk filler) (or diluent) etc.

The immune (immunity) activator/immune regulator can also be used in the form of food and drink (food and/or drink).

Examples of the above medicine include an antitumor agent, an anti-infective agent, an antiviral agent, an anti-autoimmune disease agent, an anti-diabetes agent and an anti-allergy agent, as well as pharmaceutical preparations for digestive organ diseases (therapeutic agents for irritable bowel syndrome (IBS), inflammatory bowel disease (IBD), constipation, diarrhea and the like).

Another aspect of the present invention lies in food and drink (food and/or drink) characterized by comprising any superfine particles described above (superfine particles of the present invention). The content of the superfine particles is not limited, and the content of the superfine particles in food and drink such as health foods is preferably about (approximately) 0.01 to 80% by weight, more preferably about (approximately) 0.05 to 20% by weight in terms of the whole sugar thereof.

The food and drink of the present invention can be used as health foods, functional foods, health drinks, functional drinks etc. The food and drink are particularly suitable for patients with diseases such as cancers, microbial infectious diseases, viral infectious diseases, autoimmune diseases, diabetes, allergic diseases, and digestive organ diseases (irritable bowel syndrome (IBS), inflammatory bowel disease (IBD), constipation, diarrhea and the like).

A still other aspect of the present invention lies in a superfine particle(s)-containing composition characterized by comprising an aqueous solution having the superfine particles of the present invention dispersed therein, that is, an aqueous solution (dispersion etc.) comprising the superfine particles.

The dispersion etc. can be used in a pharmaceutical composition (pharmaceutical preparation) in the same manner as described above. In this case, the pharmaceutical composition can contain a pharmaceutically acceptable carrier or excipient (bulking filler).

The dispersion etc. can be used in food and drink (food and/or drink) in the same manner as described above. In this case, the food and drink (food and/or drink) can contain (comprise) the composition in an amount of 0.05 to 5% by weight in terms of (based on) the whole sugar thereof.

The food and drink of the present invention can be used as health foods, functional foods, health drinks, functional drinks etc. The food and drink are particularly suitable for patients with diseases such as cancers, microbial infectious diseases, viral infectious diseases, autoimmune diseases, diabetes, allergic diseases, and digestive organ diseases (irritable bowel syndrome (IBS), inflammatory bowel disease (IBD), constipation, diarrhea and the like).

With respect to the content of the components in the composition, the composition can contain sugar (s) in an amount of preferably 1 to 20000 mg, more preferably 10 to 1000 mg and dispersant(s) in an amount of preferably 1 to 20000 mg, more preferably 10 to 1000 mg per 100 g of the composition.

The superfine particle(s) -containing composition is not particularly limited insofar as it is a composition comprising the superfine particles of a component derived from a mushroom, β-glucan etc. The composition includes preferably the aqueous solution containing the superfine particles of a component derived from a mushroom or β-glucan and a dispersant, more preferably the superfine particles prepared by mixing the aqueous solution containing the component(s) with a dispersant, the aqueous solution wherein the superfine particles are dispersed and the like.

Another aspect of the present invention lies in a process for producing the superfine particles characterized in that a component selected from a component derived from a mushroom and β-glucan, that is, a component derived from a mushroom or β-glucan, is subjected to a step of superfine pulverization, for example, a process for producing superfine particles which comprises subjecting a mushroom to a step of extraction with water and then subjecting the resulting aqueous extract to a step of superfine pulverization.

In particular, the superfine particles containing particles having an average particle diameter of 10 μm or less can be produced by filtering a water or hot-water extract of a mushroom through a filter paper etc. to give an aqueous mushroom extract-containing solution (an aqueous solution containing an extract of a mushroom) and then mixing a dispersant therewith preferably under stirring. In this case, it is possible to use not only an aqueous solution containing aggregates obtained by filtering a water or hot-water extract of a mushroom as the aqueous mushroom extract-containing solution (aqueous solution containing the extract thereof) through e.g. a filter paper and then concentrating and/or cooling the filtrate, but also the aqueous filtrate as it is from the filtration which will, upon being concentrated and/or cooled, give the aggregates, as described above. In case of the β-glucan, the desired particles can also be prepared in the same manner as described above.

The step of superfine pulverization can comprise a step of preparing particles having an average particle diameter of 10 μm or less by mixing a dispersant with an aqueous solution containing a component selected from a component derived from a mushroom and β-glucan, for example with an aqueous solution containing a component derived from a mushroom, and the step of superfine pulverization can comprise a step of preparing particles having an average particle diameter of 1 μm or less by finely pulverizing treatment step, for example a step of treatment with (by) a high-pressure emulsifier.

By this method, the superfine particles preferably having an immune activating activity and/or an immune regulating activity can be obtained.

A further still other aspect of the present invention lies in a process for producing a composition comprising (containing) the superfine particles, characterized by subjecting a component selected from a component derived from a mushroom and β-glucan, for example a component derived from a mushroom, to a step of superfine pulverization.

This process can be carried out according to the process for producing the superfine particles described above. The step of superfine pulverization can similarly use a step of preparing particles having an average particle diameter of 10 μm or less by mixing a dispersant with an aqueous solution containing a component selected from a component derived from a mushroom and β-glucan or a step of preparing particles having an average particle diameter of 1 μm or less by finely pulverizing treatment step, for example a step of treatment with (by) a high-pressure emulsifier. Further, the component selected from a component derived from a mushroom and β-glucan may be a water or hot water extract obtained by subjecting a mushroom to a step of extraction with water or hot water, and the aqueous solution containing a mushroom-derived component may be an aqueous solution containing a mushroom extract (an extract of a mushroom) obtained by filtering a water or hot water extract of a mushroom thorough a filter paper etc. On one hand, in case of the β-glucan extract it can also be prepared in an analogous manner.

By this method, the dispersion preferably having an immune activating activity and/or an immune regulating activity and the like can be obtained.

The average particle diameter in the methods described above refers to the average particle diameter of the particles measured (determined) in the form of a dispersion in water as described above.

Another aspect of the present invention lies in a method of activating immunity or a method of regulating immunity characterized by ingesting or administering the superfine particles of the present invention into a living body, and is extremely useful for treatment, amelioration, prevention from enlargement, prophylaxis, etc. of diseases such as tumors, infectious disease, viral infections, autoimmune diseases, diabetes, allergic diseases, and digestive organ diseases (irritable bowel syndrome (IBS), inflammatory bowel disease (IBD), constipation, diarrhea and the like) and the like.

In the ingestion or administration forms, it is possible to use the immune activator and the immune regulator etc.; or the antitumor agent, anti-infective agent, antiviral agent, anti-autoimmune disease agent, anti-diabetes agent and anti-allergy agent, as well as pharmaceutical preparations for digestive organ diseases (therapeutic agents for irritable bowel syndrome (IBS), inflammatory bowel disease (IBD), constipation, diarrhea and the like) and the like, as described above. In particular, the superfine particles can be used preferably in the form of the pharmaceutical composition and the food and drink (food and/or drink) described above.

A still other aspect of the present invention lies in a use of the novel superfine particles in (for) the immune activator or the immune regulator or production thereof; or a use of the superfine particles in (for) the antitumor agent, anti-infective agent, antiviral agent, anti-autoimmune disease agent, anti-diabetes agent and anti-allergy agent, as well as pharmaceutical preparations for digestive organ diseases (therapeutic agents for irritable bowel syndrome (IBS), inflammatory bowel disease (IBD), constipation, diarrhea and the like) and the like, and further in a use of the superfine particles in (for) production of pharmaceutical preparations.

The immune activator and the immune regulator, or the antitumor agent, anti-infective agent, antiviral agent, anti-autoimmune disease agent, anti-diabetes agent and anti-allergy agent, as well as pharmaceutical preparations for digestive organ diseases (therapeutic agents for irritable bowel syndrome (IBS), inflammatory bowel disease (IBD), constipation, diarrhea and the like) and the like are as described above, and preferable examples thereof include the form of the pharmaceutical composition or the form used for the food and drink (food and/or drink), as described above.

Brief Description of Drawings

[Figure 1]

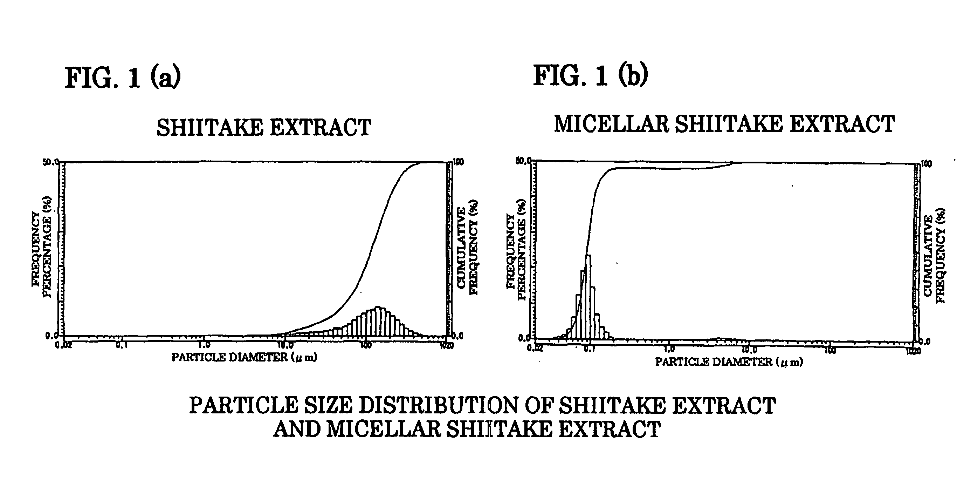

Fig. 1 shows the particle size distribution of a shiitake extract and a micellar shiitake extract in Example 1.

In the treatment of the shiitake extract with an emulsifier (emulsifying agent) [conversion into micelles] in Example 1, the particle size distributions of the shiitake extract (left: 1-a) and the micellar shiitake extract (product of the present invention) (right: 1-b) are shown. The particle diameter (μm) is shown on the abscissa, the frequency percentage (%) of the particles for the bar graph on the ordinate (left), and the cumulative frequency percentage (%) of the particles for the curve on the ordinate (right).

[Figure 2]

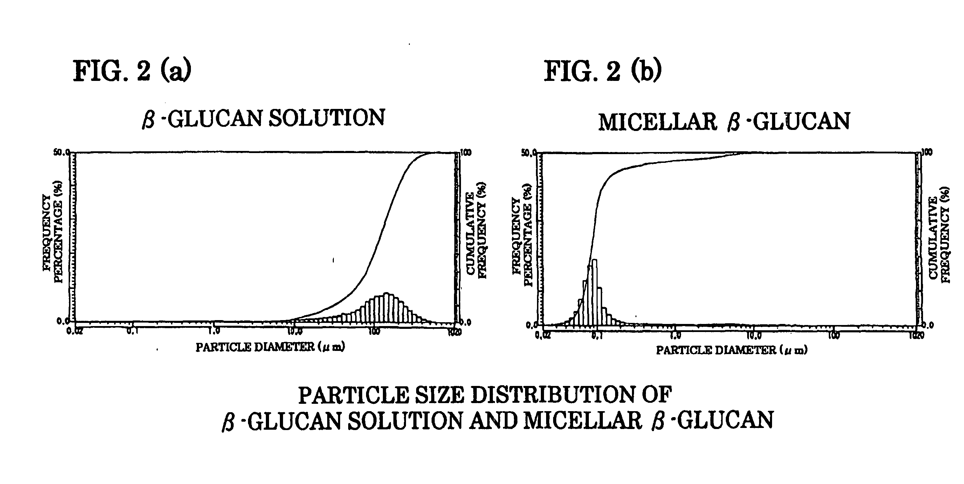

Fig. 2 shows the particle size distribution of a β-glucan solution and micellar β-glucan in Example 2.

In the treatment of the β-glucan with an emulsifier (emulsifying agent) [conversion into micelles] in Example 2, the particle size distributions of the β-glucan solution (left: 2-a) and the micellar β-glucan (product of the present invention) (right: 2-b) are shown. The particle diameter (μm) is shown on the abscissa, the frequency percentage (%) of the particles for the bar graph on the ordinate (left), and the cumulative frequency percentage (%) of the particles for the curve on the ordinate (right).

Mode for Carrying Out Invention

Hereinafter, the mode for carrying out the invention is described in more detail. Preferable and typical examples of the present invention are mainly described, but the present invention is not limited to such preferable and typical examples.

(Superfine particles; superfine particles of a mushroom extract, a β-glucan extract, etc.) .

First, the superfine particles of the present invention are described by referring mainly to the production of the superfine particles of a mushroom extract.

In the present invention, the type of mushroom is not particularly limited. Further, the site used in extraction is not particularly limited either. Edible mushrooms can be used. Typical examples include, but are not limited to, the followings.

The mushroom in the present invention refers to fungi capable of forming fruit body.

Lentinus edodes

Pleurotus ostreatus

Pholiota nameko

Flammulina velutipes

Tricholoma matsutake

Lyophyllum shimeji

Schizophyllum commune

Crepidotus variabilis

Lyophyllum ulmarium

Grifola umbellata

G. frondosa

Coriolus versicolor

Fomes fomentarius

Volvavella volvacea

Auricularia aurcula-judae

Ganoderma lucidum

G. applanatum

Fomitopsis pinicola

Dictyophora indusiata

Sparassis crispa

Agaricus blazei

Peziza vesiculosa

The used site of the mushroom is not particularly limited to special regions such as fruit body, mycelium etc. as described above. The components of a raw mushroom are varied depending on the type of mushroom, and for example, a shiitake fruit body is composed of about 90% (by weight) water, about 5% (by weight) sugar, about 2% (by weight) protein, about 1% (by weight) fiber and about 2% (by weight) other components. Accordingly, the active (efffective) ingredient in the present invention is superfine particles of non-water components extracted with water (hot water etc.).

On one hand, β-glucan is not particularly limited either, and may be a component derived from a mushroom, a component derived from yeasts, a component derived from fungi, a component derived from bacterium (bacteria), a component derived from plant(s), etc.

It is not particularly difficult to obtain an extract of a mushroom-derived component or β-glucan. For example, a mushroom may be used in extraction with water such as hot water etc. In the case, the extraction step can be easily carried out by subjecting its milled material to the step of extraction with hot water. When hot water is used, a temperature of about 60 to 100°C or so is used. A filtrate obtained by a filter paper (filter paper can be selected as necessary without particular limitation to its type) etc. after the extraction step, even whether the filtrate is a suspension containing finely pulverized particles or a solution containing aggregates obtained by further concentrating, cooling and the like the suspension, falls under the scope of the extract in the present invention.

The extract in the present invention may be a component (which may not necessarily be completely dissolved) contained in water in the extraction step with water (hot water etc.) described above, and therefore, the filtrate obtained by filtration through a filter paper etc. after the extraction step, and the fine particle component (aggregates) coagulated from the extract solution by concentration, cooling etc. also fall under the scope of this extract.

As the extracting solvent, it is possible to use not only water but also other organic solvents, but the extracting solvent is preferably water alone or a mixed solution of water and a small amount of an organic solvent, and as a matter of course, the extraction with such a water-containing solution also falls under the scope of the water extraction in the present invention. Further, even if an acid, an alkali or an inorganic substance is contained in the extracting solvent or added thereto if necessary in such a range that the amount of a mushroom extract is not adversely affected, there is no problem.

By further subjecting the extracted component to a step of superfine pulverization, the superfine particles having an immune activating activity or an immune regulating activity can be produced.

Hereinafter, the production of the superfine particles of the present invention is described in more detail by reference to preferable examples.

In an extract of a mushroom with hot water etc., for example in an extract obtained by extraction, then filtering the hot extract (filtration through Celite etc.) and cooling the filtrate or concentrating and then cooling the filtrate, aggregates having an average particle diameter of 100 μm or more are coagulated. The aggregates are considered to be those formed by aggregation of polysaccharides such as β-glucan or peptidoglycan etc. in the extract. For example, when a filtrate obtained by extracting from a milled raw shiitake mushroom at 95°C for 3 to 15 hours and filtering the extract through Celite is observed, the filtrate was confirmed to be a suspension having finely pulverized particles dispersed in the filtrate. By measuring the particle diameter of this particle, it was also confirmed that the particle is an aggregate having a median diameter of about 250 μm, and the components of this particle are β-glucan, peptidoglycan etc.

When such an extract (extract containing aggregates each having an average particle diameter of 100 μm or more) is orally ingested or administered, the active (effective) ingredient in the extract is not efficiently absorbed through a mucosa in the intestinal tract and is thus not effectively utilized in the living body. According to the superfine particles of the present invention, on the other hand, the active ingredient in the extract can be efficiently absorbed or incorporated through a mucosa in the intestinal tract to induce or cause immune reaction in lamina propria mucosae.

That is, the mushroom extract solution containing aggregates obtained by filtering a hot extract of a mushroom with water (hot water etc.) and then cooling the filtrate or concentrating and cooling the filtrate is dispersed with a dispersant etc. to disperse the coagulated aggregates, whereby superfine particles having an average particle diameter minimized to preferably 10 μm or less, more preferably 1 μm or less, still more preferably 0.01 to 1 μm or so can be produced.

For superfine pulverization of the active ingredient or coagulated aggregates in the mushroom extract, a dispersant can be used in a solution containing the active ingredient of the mushroom extract contained therein, thus dispersing the coagulated aggregates, or the active ingredient of the mushroom extract contained therein can be embedded in microcapsules etc., or the coagulated aggregates can be dispersed with a dispersant and then embedded in microcapsules etc.

Whether an immune activating action is present or not can be easily confirmed by measuring an antitumor activity, an NK (natural killer) activity, delayed type hypersensitive reaction, an amount of intracellular and extracellular cytokines, an amount of antibody produced, or the like.

The method of superfine pulverization is not particularly difficult, and superfine pulverization can be effected by using, for example, a stirrer or a homogenizer and a suitable dispersant. Further, superfine pulverization can also be effected by finely pulverizing treatment with a high-pressure emulsifier, a medium mill, supersonic waves etc.

When a dispersant is used, the dispersant is not particularly limited insofar as it is a dispersant capable of dispersing the particles in a solution, and examples thereof include surfactants, polymers, sugars, sugar alcohols, glycerides, acids, bases, salts etc. The substances used also as the emulsifier (emulsifying agent) are preferably used therefor. The emulsifiers used therefor are more preferably edible emulsifiers such as lecithin, lysolecithin, bile acid etc. In the present specification (description), dispersion with an emulsifier (emulsifying agent) refers particularly to conversion into micelles (micelles formation), but the present invention is not limited thereto, and conversion into micelles with a dispersant other than an emulsifier also falls under the scope of the present invention.

The finely pulverizing step in the present invention may be carried out at any time(s) before, after and during a step for obtaining the active ingredient such as, for example, the extraction step.

When the superfine particles of the present invention are measured in the form of a dispersion in water, the superfine particles having an average particle diameter of preferably 10 μm or less, more preferably 1 μm or less, still more preferably 0.01 to 1 μm or so can be used. When used as an immune activator/immune regulator, a solution (dispersed solution) of the superfine particles treated with a dispersant, particularly a micellar solution treated with an emulsifier (emulsifying agent), is preferably used for digestion and incorporation, but the superfine particles in a dried state can also be used as an immune activator/immune regulator.

In the present invention, the method of measuring (determining) the superfine particles can be carried out by utilizing a method of measuring usual particles, particularly dispersed particles. For example, the superfine particles can be measured by a laser diffraction/scattering particle size distribution measuring method using a particle size distribution meter.

(Immune activator/immune regulator)

As described above to some degrees, the superfine particles of the present invention can be utilized as the active ingredient of an immune activator/immune regulator (immune activator/immune regulator of the present invention). A carrier or an excipient (bulking filler) (or a diluent) usable in the pharmaceutical composition or the food and drink (food and/or drink) according to the present invention can also be used. Specifically, the immune activator/immune regulator can be used as the pharmaceutical composition, the food and drink (food and/or drink) (health foods etc.) and the like.

Whether an immune activating action or immune regulating action is present or not can be easily confirmed by measuring e.g. an antitumor activity, an NK activity, delayed type hypersensitive reaction, an amount of intracellular and extracellular cytokines or an amount of antibody produced.

(Pharmaceutical composition)

The pharmaceutical composition (agent; drug; pharmaceutical preparation) of the present invention is an agent (a pharmaceutical preparation) which comprises the superfine particles as described above, preferably the solution treated with a dispersant (dispersed solution), more preferably the solution containing the micellar component(s) as the active ingredient with an emulsifier (emulsifying agent) and which can be used for treatment, amelioration and prevention from enlargement, of diseases accompanying abnormalities in immunity or for prophylaxis etc. of other diseases by activating or regulating immunity, particularly systemic immunity. For example, the pharmaceutical composition can be used for an antitumor agent, an anti-infective agent, an antiviral agent, an anti-autoimmune disease agent, an anti-diabetes agent and an anti-allergy agent, as well as an agent for digestive organ diseases (therapeutic agent for irritable bowel syndrome (IBS), inflammatory bowel disease (IBD), constipation, diarrhea and the like) and the like, and used for treatment or prevention (preservation) of these various diseases.

The subject to which this agent (pharmaceutical preparation) is applied is an animal, particularly a human seeking activation or regulation of immunity, particularly systemic immunity.

One characteristic of the pharmaceutical preparation of the present invention is that an excellent effect is brought about even by oral administration; a component derived from a mushroom and β-glucan, for example an extracted mixture from a mushroom, particularly from an edible mushroom etc. can be used; and the pharmaceutical preparation is particularly excellent in safety. Accordingly, the form of administration is not particularly limited. Various forms of administration such as oral administration, parenteral administration (subcutaneous administration, intramuscular administration, nasal administration, aerosol administration etc.) can be used, and the pharmaceutical preparation can be applied widely and easily to patients seeking an immune activating action and/or an immune regulating action. The pharmaceutical preparation is suitable for safety and oral administration, and can thus be used in the form of health foods, functional foods, health drinks, functional drinks etc. described later, in order to prevent and ameliorate the intended disease.

In the present invention, the pharmaceutical preparation can be mixed or combined with other pharmaceutical component(s) (pharmaceutically active substance(s)), and insofar as a certain pharmaceutical preparation comprises the desired active ingredient in the present invention to exhibit the desired pharmacological activity (immune activating activity or immune regulating activity), such pharmaceutical preparation falls under the scope of the pharmaceutical preparation of the present invention.

In addition, the pharmaceutical preparation can further contain a wide variety of pharmacologically acceptable pharmaceutical material(s) (as adjuvant etc.) for pharmaceutical preparation. The pharmaceutical material(s) can be selected suitably depending on the form of the preparation, and examples thereof include excipients, diluents, additives, disintegrating agents, binders, coating agents, lubricants, sliding agents, lubricants (lubricant pharmaceuticals), flavorings, sweeteners, emulsifiers (emulsifying agents), solubilizers etc. Further examples of the pharmaceutical materials include magnesium carbonate, titanium dioxide, lactose, mannitol and other sugars, talc, milk protein, gelatin, starch, cellulose and derivatives thereof, animal and vegetable oils, polyethylene glycol, and solvents such as sterilized water and monovalent or polyvalent alcohols, for example glycerol.

The pharmaceutical preparation of the present invention can be prepared in various pharmaceutical forms known in the art or to be developed in the future as described above, for example in administration forms for oral administration, intraperitoneal administration, transdermal administration, inhalation administration etc. To prepare the agent (pharmaceutical preparation) of the present invention in such various pharmaceutical preparation forms, methods known in the art or to developed in the future can be suitably used.

The forms of these various pharmaceutical preparations include, for example, suitable solid or liquid pharmaceutical forms such as granules, powders, coated tablets, tablets, (micro) capsules, suppositories, syrups, juices, suspensions, emulsions, dropping agents, injection solutions, preparations prolonging release of the active agent, etc.

As a matter of course, the pharmaceutical preparation of the present invention in the pharmaceutical preparation forms illustrated above should contain a pharmaceutically effective amount of the above described component(s).

The amount of the pharmaceutical preparation of the present invention administered is selected suitably depending on the type and severeness of the intended disease, the form of the pharmaceutical preparation, etc. For example, the superfine particles of the active ingredient can be administered orally to a patient in a daily dose of preferably 1 mg to 50 g or so, more preferably 10 mg to 10 g or so, still more preferably 50 mg to 5 g or so expressed in terms of the whole sugar thereof. In the case of severer diseases, the dose can be further increased. With respect to the frequency and intervals of administration, the pharmaceutical preparation of the superfine particles can be administered once every a few days or once every day, but is usually administered for example before, between and/or after meal (or each meal) in 2 to 4 divided portions several times every day. Preferably, the pharmaceutical preparation of the superfine particles is administered before meal. In the case of intravenous administration, the dose may be one tenth to hundredth (1/10 to 1/100) as small as the dose in oral administration.

(Food and drink)

Even when the food and drink (food and/or drink) of the present invention are used particularly as health foods or functional foods, the food and drink can be prepared on the basis of the above-described oral preparation by adding component(s) (including extract(s) derived from different mushroom(s)) and additives necessary for health foods or functional foods. As a matter of course, edible or nutrient ingredients etc. used in food and drink can be added if necessary and used. Usually, the superfine particles can be contained in an amount of preferably 0.01 to 80% or so by weight, more preferably 0.05 to 20% or so by weight in terms of the whole sugar therein.

Flavorings or sweeteners usable in food and drink can be used to form a solution usable in the form of drink or a form in the form of tablets, granules or capsules, or a form in a jelly or ice cream form, or one in a frozen form or the like.

The food and drink can be used for prevention not only for healthy persons but also for patients with severe to light various diseases, particularly for patients seeking systemic immunity activation or immunity regulation without limitation to patients with diseases accompanying abnormalities in immune functions. For animals other than humans, the food and drink can be applied in forms such as feed, pharmaceutical products (preparations) and pharmaceutical compositions.

(Superfine particles-containing composition)

This invention lies in an aqueous solution wherein the superfine particles of a component selected from a component derived from a mushroom and β-glucan are dispersed, and this invention(s) can be easily understood and practiced from the description for the superfine particles, the various uses thereof or the production method (process) described above. As a matter of course, the composition similar to the superfine particles can be used in various uses, particularly for a pharmaceutical composition or food and drink (food and/or drink), and the above these descriptions therefor can also be applied to this invention.

As described above, a still other aspect of the present invention lies in a method of activating immunity or a method of regulating immunity characterized by ingesting or administering the superfine particles of the present invention into a living body, and a further still other aspect of the present invention lies in a use of the superfine particles for an immune activator or an immune regulator; or uses of the superfine particles for an antitumor agent, anti-infective agent, antiviral agent, anti-autoimmune disease agent, anti-diabetes agent and anti-allergy agent, as well as pharmaceutical preparations for digestive organ diseases (therapeutic agents for irritable bowel syndrome (IBS), inflammatory bowel disease (IBD), constipation, diarrhea and the like) and the like, and further in a use thereof for production of the pharmaceutical products (preparations).

These inventions can be easily carried out on the basis of the above description for the immune activator or the immune regulator, or the descriptions for the antitumor agent, anti-infective agent, antiviral agent, anti-autoimmune disease agent, anti-diabetes agent and anti-allergy agent, as well as pharmaceutical preparations for digestive organ diseases (therapeutic agents for irritable bowel syndrome (IBS), inflammatory bowel disease (IBD), constipation, diarrhea and the like) and the like or the descriptions for the pharmaceutical composition, the food and drink (food and/or drink) etc., or on the basis of preferable embodiments (Examples) described later, or by reference to known techniques if necessary.

This invention (product of the present invention) can be used not only in humans but also in other animals as described above, and for example, the product of the present invention is also useful as feed for animals in livestock industry (cattle, pigs, sheep, horses, birds etc.), pets (dogs, cats etc.) or fishes in fisheries and raising industry (bony fishes, crustaceans etc.) or as additives to be added to feed or as pharmaceutical preparations or pharmaceutical compositions.

Preferable Embodiments

Hereinafter, the present invention is described in more detail by reference to Production Examples, Examples and Comparative Examples, but the present invention is not limited to these examples.

(Example 1)

(Method of extraction from shiitake mushroom)

About 4 L water was added to per kg (in terms of raw shiitake mushrooms) of shiitake mushrooms, and the mushrooms were disrupted with a colloid mill etc. (volume of the solution after disruption: about 6 L) and boiled at 95°C for 15 hours under heating under reflux to prevent evaporation of water, and the resulting extract was filtered. The filtrate was concentrated at 60°C under reduced pressure to give about 1 L concentrate. The extract was analyzed for its sugars by a phenol-sulfuric acid method, indicating that the content of sugars in the extract was a 20 mg/ml concentration (this extract may be referred to hereinafter as "extract of shitake"; or "shitake extract").

(Treatment of the shiitake extract with an emulsifier (emulsifying agent) [conversion into micelles])

Lecithin (SLP-PC70) manufactured by Tsuru Lecithin Kogyo Co., Ltd. was added to deionized water to prepare a solution containing lecithin at the same concentration as that of the whole sugar in the shiitake extract. To the lecithin solution was added the same volume of the above shiitake extract, and the mixture was stirred under vacuum (vacuum pressure: -60 cmHg; number of revolutions (rotation frequency) of an anchor mixer: 50 rpm; number of revolutions of a homomixer: 15,000 rpm) by an Agi homomixer 2M-2 model manufactured by Tokushu Kika Kogyo Co., Ltd., to prepare a preliminary micellar solution. The resulting preliminary emulsified solution was subjected to high-pressure emulsification treatment (emulsification pressure 1,500 kgf/cm$^2$) with a high-pressure emulsifier H11 model in a 2-step handle system, manufactured by Sanwa Kikai Co., Ltd., to prepare a micellar solution of the shiitake extract having a median diameter of about 100 nm (micellar shiitake extract: the product of the present invention). Measurement of the median diameter was carried out by a laser diffraction/scattering particle size distribution measurement method using an LA-910 particle size distribution meter manufactured by Horiba Seisakusho Co., Ltd.

The measurement results of the particle size distribution of the shiitake extract and the micellar shiitake extract are shown in Fig. 1. The results indicated that the component in the shiitake extract had a median particle diameter of about 120 $\mu$m, and by treatment of the component in the shiitake extract with an emulsifier (emulsifying agent) ("conversion into micelles"), the component could be converted into superfine particles having a median particle diameter of about 0.09 $\mu$m.

(Example 2)

(Preparation of $\beta$-glucan solution)

Purification of $\beta$-glucan from raw shiitake mushrooms was carried out according to the method of Chihara et al. (Cancer Res., 30, 2776 (1970)). That is, fruit bodies of raw shiitake mushrooms were subjected to extraction with hot water, then repeatedly to fractionating precipitation with ethanol, to fractionating precipitation with cetyltrimethyl ammonium hydroxide, to fractionating elution with acetic acid and to fractionating elution with sodium hydroxide followed by removal of protein, whereby white powder of $\beta$-glucan was obtained. The resulting white powder was suspended in distilled water, homogenated and subjected to high-temperature and high-pressure treatment (121°C, 20 minutes) in an autoclave to prepare 2 mg/ml $\beta$-glucan solution.

(Treatment of $\beta$-glucan with an emulsifier (emulsifying agent) [conversion into micelles])

Lecithin (SLP-PC70) manufactured by Tsuru Lecithin Kogyo Co., Ltd. was added to deionized water to prepare a solution containing lecithin dissolved therein at a concentration of 8 mg/ml. To the lecithin solution was added the same volume of the above $\beta$-glucan solution, and the mixture was subjected to high-pressure emulsification treatment (emulsification pressure 1,500 kgf/cm$^2$) with a high-pressure emulsifier H11 model in a 2-step handle system, manufactured by Sanwa Kikai Co., Ltd., to prepare a micellar solution of $\beta$-glucan having a median diameter of about 100 nm (micellar $\beta$-glucan: the product of the present invention). Measurement of the median diameter was carried out by a laser diffraction/scattering particle size distribution measurement method using an LA-910 particle size distribution meter manufactured by Horiba Seisakusho Co., Ltd.

The measurement results of the particle size distribution of the $\beta$-glucan solution and the micellar $\beta$-glucan are shown in Fig. 2. The results indicated that $\beta$-glucan in the $\beta$-glucan solution formed aggregates having a median particle diameter of about 120 $\mu$m, and by treatment thereof with an emulsifier (emulsifying agent) ("conversion into micelles"), the aggregates could be converted into superfine particles having a median particle diameter of about 0. 09 $\mu$m.

(Example 3)

(Example using an S180 subcutaneous inoculation model)

(Test method)

Sarcoma 180 tumor cells maintained by intraperitoneal injection in ICR mice (female, 4-week-old) were collected in the form of ascitic fluid and prepared at a density of $3\times10^7$ cells/ml with physiological saline. This cell suspension was subcutaneously inoculated in a volume of 0.1 ml/mouse through a 25 G needle into a right groin of ICR mice (female, 4-week-old).

On the next day, the mice were grouped (7 mice/group) depending on their weight, and were identified, and then administrations of the extract of shiitake (shiitake extract) and the micellar solution of the extract of shiitake (micellar shiitake extract) were initiated. In this administration, the extract was orally administered (one time/day) 5 times per week, and the administration was conducted 10 times in total. The dose for each administration was as follows: the extract (sample) adjusted to a concentration of 1 mg/ml was given in a dose of 0.2 ml/mouse to a 10 mg (in terms of whole sugar) /kg administration group, and the extract (sample) adjusted to 10 mg/ml was given in a dose of 0.2 ml/mouse to a 100 mg (in terms of whole sugar)/kg administration group.

The number in the brackets after "Shiitake extract" and "Micellar shiitake extract", shown in the item "Administration group" in Tables 1 and 2, is the administration dose of sample in terms of whole sugar (unit: mg/kg). In this example, the particles (the superfine particles) subjected to superfine pulverization treatment with an emulsifier (emulsifying agent) are referred to as the micellar shiitake extract.

The tumor size and the body weight were measured once per week. From the tumor size, the tumor weight was calculated according to the following formula:

$$\text{Tumor weight (mg)} = \text{tumor minimum diameter(mm)}^2 \times \text{tumor maximum diameter(mm)} \div 2$$

Further, the host weight was also calculated from the tumor weight and body weight.

$$\text{Host weight (g)} = \text{body weight (g)} - \text{tumor weight (g)}$$

From the tumor weight, the degree of inhibition of tumor growth was calculated.

$$\text{Degree of inhibition of tumor growth (\%)} = (1 - \text{tumor weight of administration group} \div \text{tumor weight of non-treatment group}) \times 100$$

From the degree of inhibition of tumor growth in each week and the number of tumor bearing mice on the 35th day after inoculation of the tumor, the pharmaceutical effect on this model was evaluated. The results of the inhibitory effect of the micellar shiitake extract on the tumor growth are shown in Tables 1 and 2.

Table 1 Tumor weight (g)

| Administration group | Day16 | | | |
|---|---|---|---|---|
| | Average (g) | S.E. | t-test | M test |
| Non-treatment group | 2.493 | 0.246 | - | - |
| Emulsifier only | 2.817 | 0.401 | N.S. | N.S. |
| Shiitake extract (10) | 1.844 | 0.363 | N.S. | N.S. |
| Shiitake extract (100) | 2.712 | 0.522 | N.S. | N.S. |
| Micellar shiitake extract (10) | 1.507 | 0.163 | p<0.01 | p<0.01 |

(continued)

| Administration group | Day16 | | | |
|---|---|---|---|---|
| | Average (g) | S.E. | t-test | M test |
| Micellar shiitake extract (100) | 1.744 | 0.394 | N.S. | N.S. |
| Day 16: 16th day after inoculation of the tumor<br>t-test: Student's t-test: t-test of each group as compared with the non-treatment group was conducted.<br>M test: Mannwhitney-U test: Rank test of each group as compared with the non-treatment group was conducted.<br>N.S., not significant; p<0.01, significant. | | | | |

In the group administered the micellar shiitake extract, the tumor growth was inhibited gradually as compared with the non-treatment group during the administration period, and on Day 16 (2nd day after the administration was finished), the tumor growth was inhibited significantly (p<0.01) in the group orally administered the micellar shiitake extract in an administration dose of 10 mg (in terms of whole sugar)/kg.

Table 2 Inhibitory effect on tumor growth

| Administration group | Degree of inhibition of tumor growth (%) |
|---|---|
| | Day16* |
| Non-treatment group | - |
| Emulsifier only | -13.0 |
| Shiitake extract (10) | 26.0 |
| Shiitake extract (100) | -8.8 |
| Micellar shiitake extract (10) | 39.6 |
| Micellar shiitake extract (100) | 28.8 |
| *: 16th day after transplant of the tumor | |

As is evident from the results shown above, it was confirmed that the superfine particles of the present invention exhibit the desired pharmaceutical effect as compared with the conventional products.

(Example 4)

(Quantification of β-glucan)

To quantify β-glucan in the shiitake extract, the β-glucan purified in Example 2 was dissolved in a sodium hydroxide aqueous solution (1→25) and then precipitated (coagulated) with methanol, and this procedure was repeated twice, and then the sample was washed with methanol and acetone and dried under reduced pressure (40°C, 15 hours) to give standard β-glucan containing 0.03% or less nitrogen and 1.5% or less loss on drying.

For quantitative determination of the β-glucan in the shiitake extract, using the previously purified standard β-glucan, β-glucan concentration was quantitatively determined by application of the method of Sasaki et al. (Gann, 67(2) 191-5 (1976)). That is, the shiitake extract and the standard β-glucan were prepared in a sodium hydroxide aqueous solution (2 g/dl) respectively, and a Congo red solution (10 mg/dl) and phosphoric acid (2.5 g/dl) were added thereto, and utilizing the shift of the local maximum absorption wavelength of Congo red by β-glucan, the β-glucan was quantitatively determined by measuring the absorbance at 535 nm with a spectrophotometer.

The shiitake extract quantified for β-glucan level was used to prepare a micellar shiitake extract (lecithin concentration, 0.8 mg/ml) containing β-glucan at a concentration of 0.2 mg/ml in the same manner as in Example 1. The β-glucan solution was also subjected to emulsification treatment with lecithin to prepare a micellar β-glucan solution containing β-glucan at a concentration of 0.2 mg/ml in the same manner.

(Example 5)

(Inhibitory effect of the micellar β-glucan on tumor growth)

The inhibitory effects of the micellar shiitake extract, the micellar β-glucan and the β-glucan solution prepared above on tumor growth were examined in the same manner as in Example 3. That is, sarcoma 180 tumor cells maintained by intraperitoneal injection in ICR mice (female, 4-week-old) were collected in the form of ascitic fluid and prepared at a density of $3 \times 10^7$ cells/ml with physiological saline. This cell suspension was subcutaneously inoculated in a volume of 0.1 ml/mouse through a 25 G needle into a right groin of ICR mice (female, 4-week-old).

On the next day, the mice were grouped (7 mice/group) depending on their weight, and were identified, and then administrations of the micellar shiitake extract, the micellar β-glucan and the β-glucan were initiated. In this administration, the extract was orally administered (one time/day) 5 times per week, and the administration was conducted 10 times in total. The dose for each administration was as follows: the sample adjusted to a β-glucan concentration of 0.2 mg/ml was given in a dose of 0.1 ml/mouse to an each 1 mg (in terms of β-glucan)/kg administration group.

The number in the brackets after "Micellar shiitake extract", "Micellar β-glucan" and "β-Glucan", shown in the item "Administration group" in Tables 3 and 4, is the administration dose in terms of β-glucan (unit: mg/kg). In this example, the particles (the superfine particles) subjected to superfine pulverization treatment with an emulsifier (emulsifying agent) are referred to as the micellar shiitake extract and micellar β-glucan.

Table 3 Tumor weight (g)

| Administration group | Day16 | | | |
|---|---|---|---|---|
| | Average (g) | S.E. | t-test | M test |
| Non-treatment group | 3.011 | 0.323 | - | - |
| Micellar shiitake extract (1) | 1.496 | 0.343 | p<0.01 | p<0.01 |
| Micellar β-glucan (1) | 1.468 | 0.256 | p<0.01 | p<0.01 |
| β-Glucan (1) | 2.041 | 0.391 | N.S. | N.S. |
| Day 16: 16th day after transplant of the tumor<br>t-test: Student's t-test: t-test of each group as compared with the non-treatment group was conducted.<br>M test (assay) : Mannwhitney-U test (assay) : Rank test of each group as compared with the non-treatment group was conducted. N.S., not significant; p<0.01, significant. | | | | |

In the groups administered the micellar shiitake extract and the micellar β-glucan respectively i.e. the groups administered the superfine particles with superfine pulverization treatment, the tumor growth was inhibited gradually as compared with the non-treatment group during the administration period, and on Day 16 (2nd day after the administration was finished), the tumor growth was inhibited significantly (p<0.01) in the group orally administered the micellar particles in a dose of 1 mg/kg in terms of β-glucan.

Table 4 Inhibitory effect on tumor growth

| Administration group | Degree of inhibition of tumor growth (%) |
|---|---|
| | Day 16* |
| Non-treatment group | - |
| Micellar shiitake extract (1) | 50.3 |
| Micellar β-glucan (1) | 51.2 |
| β-Glucan (1) | 32.2 |
| *: 16th day after transplant of the tumor | |

As is evident from the results shown above, it was confirmed that the superfine particles of the present invention (micellar shiitake extract and micellar β-glucan) exhibit the desired pharmaceutical effect as compared with the conventional product (β-glucan solution).

(Example 6)

(Patho-histological investigation: activation of mucosal immunity in the intestinal tract)

The activating effect of the micellar shiitake extract on mucosal immunity in the intestinal tract was investigated in the same manner as in Example 3. That is, sarcoma 180 tumor cells maintained by intraperitoneal injection in ICR mice (female, 4-week-old) were collected in the form of ascitic fluid and prepared at a density of $3 \times 10^7$ cells/ml with physiological saline. This cell suspension was subcutaneously inoculated in a volume of 0.1 ml/mouse through a 25 G needle into a right groin of ICR mice (female, 4-week-old).

On the next day, the mice were grouped (3 mice/group) depending on their weight, and were identified, and then administrations of an emulsifier (lecithin) only, the emulsifier plus the shiitake extract (not subjected to superfine pulverization treatment [conversion into micelles]), and the micellar shiitake extract were initiated. In these administrations, the extract was orally administered (one time/day), 5 times per week, and the administration was conducted 10 times in total. The dose for each administration was as follows: lecithin as the emulsifier at a concentration of 2 mg/ml was given in a dose of 0.1 ml/mouse to a group administered the emulsifier; the emulsifier plus the shiitake extract were given at a concentration of 0.2 mg/ml β-glucan (lecithin concentration: 2.0 mg/ml) in a dose of 0.1 ml/mouse to a group administered the emulsifier plus the shiitake extract in a dose of 1 mg/kg in terms of β-glucan; and the micellar shiitake extract adjusted to a concentration of 0.2 mg/ml β-glucan (lecithin concentration: 2 mg/ml) was given in a dose of 0.1 ml/mouse to a group administered the micellar shiitake extract in a dose of 1 mg/kg in terms of β-glucan or in a dose of 0.3 ml/mouse to a group administered the micellar shiitake extract in a dose of 3 mg/kg in terms of β-glucan.

On the second day after the administrations were finished, small intestines with Peyer patches were removed from each group and normal mice (3 animals) and then fixed in 10% formalin and embedded in paraffin to prepare paraffin sections which were then deparaffined and stained with hematoxylin/eosin, and accumulation of mononuclear cells (immunocompetent cells) in a lamina propria mucosae in the intestinal tract was observed under a microscope.

The number in the brackets after "Shiitake extract" and "Micellar shiitake extract", shown in the item "Administration group" in Table 5, is the administration dose in terms of β-glucan (unit: mg/kg). In this example, the particles (the superfine particles) subjected to superfine pulverization treatment with an emulsifier (emulsifying agent) are referred to as the micellar shiitake extract.

Table 5 Number of areas of mononuclear cell accumulation in the lamina propria mucosae in the intestinal tract (percentage (%) : number of areas per number of villus.)

| Administration group | Percentage (%) of mononuclear cells accumulation per villus | | t-test |
|---|---|---|---|
| | Average (%) | S.D. | |
| Normal mice[1] | 0.00 | 0.00 | - |
| Non-treatment group | 0.31 | 0.27 | - |
| Emulsifier only | 0.24 | 0.41 | N.S. |
| Emulsifier + shiitake extract (1)[2] | 0.35 | 0.60 | N.S. |
| Micellar shiitake extract (1) | 1.33 | 0.73 | P<0.1 |

(continued)

| Administration group | Percentage (%) of mononuclear cells accumulation per villus | | t-test |
| --- | --- | --- | --- |
| | Average (%) | S.D. | |
| Micellar shiitake extract (3) | 1.34 | 0.08 | P<0.01 |

The number of villi (100 to 300 villi/mouse) was measured under a microscope, and the number of area of mononuclear cell accumulation in the lamina propria mucosae in the intestinal tract was measured, and the percentage of number of accumulated areas per villus was calculated.
[*1]: Area of accumulation of mononuclear cells was not observed in the lamina propria mucosae in the intestinal tract in the normal mice.
[*2]: The shiitake extract was merely added to the emulsifier (lecithin) and not subjected to superfine pulverization treatment ("conversion into micelles").
t-test: Student's t-test: t-test of each group as compared with the non-treatment group was conducted.
N.S., not significant; p<0.1, falsely significant; p<0.01, significant.

Tissues of small intestines in the normal mice, the untreated group in the tumor bearing mice (non-treatment group), the group treated with the emulsifier (group administered orally 10 mg/kg lecithin), the group administered orally the emulsifier plus the shiitake extract in a dose of 1 mg/kg in terms of β-glucan, and the groups administered orally the micellar shitake extract in doses of 1 mg/kg and 3 mg/kg in terms of β-glucan were observed under a microscope, and as a result, the groups administered the micellar shiitake extract as compared with the non-treatment group showed accumulation of mononuclear cells (lymphocytes, macrophages) in a lamina propria mucosae in the small intestine falsely significantly (p<0.1) in the group given 1 mg/kg, and significantly (p<0.01) in the group given 3 mg/kg. In the groups other than the groups administered the micellar shiitake extract, there was no difference from the non-treatment group. Accordingly, it is considered that immune reaction is induced (activated) in the lamina propria mucosae in the intestinal tract by orally administering the micellar shiitake extract.
As is evident from the results shown above, it was confirmed that the superfine particles of the present invention (micellar shiitake extract) exhibit the desired pharmaceutical effect as compared with the conventional product (shiitake extract).

(Example 7)

(Activation of systemic immunity: delayed type hypersensitive reaction)

To investigate the activation of systemic immune reaction specifically on tumor antigen, the delayed type hypersensitive reaction was evaluated. The method of evaluating the delayed type hypersensitive reaction is described.
The tumor was transplanted in the same manner as in Example 3. That is, sarcoma 180 tumor cells maintained by intraperitoneal injection in ICR mice (female, 4-week-old) were collected in the form of ascitic fluid and prepared at a density of $3 \times 10^7$ cells/ml with physiological saline. This cell suspension was subcutaneously inoculated in a volume of 0.1 ml/mouse through a 25 G needle into a right groin of ICR mice (female, 4-week-old).
On the next day, the mice were grouped (7 mice/group) depending on their weight, and were identified, and then administration of the micellar shiitake extract, the micellar β-glucan and the β-glucan solution was initiated. In this administration, each sample was orally administered (one time/day) 5 times per week, and the administration was conducted 9 times in total. The dose for each administration was as follows: each of the micellar shiitake extract and the micellar β-glucan, adjusted to a concentration of 0.2 mg/ml in terms of β-glucan (lecithin concentration: 0.8 mg/ml), was given in a dose of 0.1 ml/mouse to a group administered that in a dose of 1 mg/kg in terms of β-glucan. The β-glucan solution, adjusted to 0.2 mg/ml in a concentration of β-glucan, was administered in a dose of 0.1 ml/mouse to a group administered the solution in a dose of 1 mg/kg β-glucan.
Seven mice each of the non-treatment group, the group administered the micellar shiitake extract, the group administered the micellar β-glucan, the group administered the β-glucan solution, and 3 normal mice, were subjected to a delayed type hypersensitivity (DTH) test. That is, on the 9th day after the tumor inoculation (that is, on the 8th day after the administration was initiated) , 50 μl physiological saline was administered as the control into the right foot pad, while 50 μl tumor antigen solution obtained from sarcoma 180 cells by a 3 M KCl solubilization method or a freezing and thawing method (cellular suspension at a density of $5 \times 10^7$ cells/ml was treated) was administered into the left foot pad, and 24 hours later, the thickness of each of the right foot pad and the left foot pad was measured, and the swelling of the foot was calculated from the following equation, to evaluate the DTH reaction.

$$\text{Swelling of foot (mm)} = \text{thickness of left foot pad (mm)} - \text{thickness of right food pad (mm)}$$

The number in the brackets after "Micellar shiitake extract", "Micellar β-glucan" and "β-Glucan solution", shown in the item "Administration group" in Table 6, is the dose of the administered sample in terms of β-glucan (unit: mg/kg). In this example, the superfine particles subjected to superfine pulverization treatment with an emulsifier (emulsifying agent) are referred to as the micellar shiitake extract and the micellar β-glucan.

Table 6 Delayed type hypersensitive reaction; swelling of foot (mm)

| Administration group | Swelling of foot (mm) | | t-test |
|---|---|---|---|
| | Average (mm) | S.D. | |
| Normal mice[*1] | 0.08 | 0.05 | - |
| Non-treatment group | 0.01 | 0.05 | - |
| Micellar shiitake extract (1) | 0.23 | 0.25 | p<0.05 |
| β-Glucan solution (1) | 0.08 | 0.11 | N.S. |
| Micellar β-glucan (1) | 0.17 | 0.08 | p<0.01 |
| *1: The normal mice did not cause DTH reaction because they did not undergo tumor inoculation (antigen sensitization). t-test: Student's t-test: t-test of each group as compared with the non-treatment group was conducted. N.S., not significant; p<0.05, significant; p<0.01, significant | | | |

As shown in Table 6, the swelling of foot pads in the normal mice, the non-treatment group, and the group administered the β-glucan solution was scarcely observed, while significant swelling of foot pads in the group administered the micellar shiitake extract and the group administered the micellar β-glucan was observed significantly as compared with the non-treatment group, and it was thus confirmed that the delayed type hypersensitive reaction was induced. These results indicate that systemic immune reaction against the tumor antigen can be induced by oral administration of the micellar shiitake extract and the micellar β-glucan.

(Example 8)

(Anti-allergic effect)

The anti-allergic effect of the micellar shiitake extract was examined using NC mice i.e. model mice with atopic dermatitis. That is, twenty 8-week-old male NC mice were sensitized by applying 150 µl/mouse antigen ; picryl chloride (5% (w/v)) prepared in ethanol and acetone (4 : 1) onto their abdomens and foot pads, and from the fourth day after the sensitization, an inducing antigen ; picryl chloride (0.8% (w/v)) prepared in olive oil was applied once per week in an amount of 150 µl/mouse for 6 weeks onto the back and ears (internal and external sides). The administration was initiated from the previous day of sensitization with the antigen and conducted once daily for 46 days. The amount of each sample administered was as follows: As the solvent control, physiological saline was orally administered in an amount of 0.1 ml/mouse into the control group (10 mice). The micellar shiitake extract, adjusted to a concentration of 0.2 mg/ml in terms of β-glucan, was orally administered in an amount of 0.1 ml/mouse. This dose in each administration corresponds to a dose of 1 mg/kg in terms of β-glucan.

For evaluation of the anti-allergic effect, sensitization with the antigen was conducted once every week, the body weight was measured, and for the degree of dermatitis, (1) pruritus/itching, (2) erythema/hemorrhage, (3) edema, (4) excoriation/erosion and (5) scaring/dryness were observed and evaluated (evaluation point: 0, asymptomatic; 1, slight; 2, moderate; 3, severe). On the 29th and 46th days after sensitization with the antigen, blood was collected from the orbital vein, serum was obtained therefrom, and the immunoglobulin E (IgE) level in the serum was measured by ELISA. Results: On the 46th day after the sensitization, the IgE level was at least 5.0 µg/ml in all 10 mice in the control group, while the IgE level in 4 mice out of 10 mice in the group administrated the micellar shiitake extract was at least 5.0 $\mu$g/ml, but the other 6 mice indicated an IgE level of less than 5.0 $\mu$g/ml, thus showing that the increase of the IgE level was significantly suppressed (Fisher's probability test: p<0.05). Further, on the 46th day after the sensitization, the total dermatitis score was 9 or more in 9 mice out of 10 mice in the control group, while the total dermatitis score was 9 or more in 5 mice out of 10 mice in the group administrated the micellar shiitake extract, and the other 5 mice indicated a total dermatitis score of less than 9, showing that the dermatitis was suppressed as compared with the control group.

As is evident from the results shown above, it was confirmed that the superfine particles of the present invention (micellar shiitake extract) exhibit the desired pharmaceutical effect (anti-allergic effect).

(Example 9)

(Anti-diabetic effect: Inhibitory effect on increase in blood sugar levels)

To investigate the effect of the micellar shiitake extract on diabetes, db/db mice i.e. model mice with type II diabetes were used to examine the effect on increase in blood sugar levels. That is, 5-week-old male db/db mice were divided into 2 groups each consisting of 9 mice, and one group were allowed water ad libitum as the control group, while the other group was given micellar shiitake extract-containing water ad libitum until the mice were 12-week-old. The concentration of the micellar shiitake extract was 0.01 mg/ml in terms of $\beta$-glucan (lecithin concentration: 0.1 mg/ml). The amount of water drunk was measured every week, to calculate the amount of orally ingested $\beta$-glucan.

For evaluation of the effect, blood was collected from the orbital vein every week, blood sugar levels were measured, and the inhibitory effect on increase in sugar blood levels was examined. The amount of orally ingested $\beta$-glucan was in the range of from 0.15 mg/mouse/day to 0.36 mg/mouse/day in the 6- to 12-week-old mice.

In the 8-week-old mice, the blood sugar levels in the control group were 504.11$\pm$50.03 mg/dl, while the blood sugar levels in the group administered the micellar shiitake extract were 406.22$\pm$75.55 mg/dl, indicating that the blood sugar levels were significantly prevented from increasing (Student's t-test: p<0.01).

As is evident from the results shown above, it was confirmed that the superfine particles of the present invention (micellar shiitake extract) exhibit the desired pharmaceutical effect (inhibitory effect on increase in blood sugar levels in diabetes).

Effect of Invention

According to the present invention, there can be provided an immune activator and an immune regulator which can improve an animal, particularly human immunocompetence, particularly a pharmaceutical composition, food and drink (health foods, functional foods etc.) having such an excellent immune activating action and/or immune regulating action and the like. Further, there can be also provided a novel substance (or a novel composition) usable as an active (effective) ingredient for such excellent products, specifically superfine particles of a component selected from a component derived from a mushroom and $\beta$-glucan, for example, superfine particles of an extract of a mushroom, preferably a water extract thereof treated with a dispersant (dispersion), particularly a micellar solution thereof obtained by treatment with an emulsifier (emulsifying agent).

According to the present invention, there are also provided a method of activating immunity or a method of regulating immunity for medical treatment, prophylaxis (prevention) etc. of various diseases, a use of the superfine particles for the immune activator or the immune regulator; or a use of the superfine particles for the antitumor agent, the anti-infective agent, the antiviral agent, the anti-autoimmune disease agent, the anti-diabetes agent and the anti-allergy agent, as well as the pharmaceutical preparations for digestive organ diseases (therapeutic agents for irritable bowel syndrome (IBS), inflammatory bowel disease (IBD), constipation, diarrhea and the like) and the like, and a use thereof for production of pharmaceutical products (preparations).

According to the present invention, there can be further provided a process for producing a mushroom-derived component and $\beta$-glucan as an efficacious ingredient by easy production means, particularly the superfine particles of the active ingredient having the immune activating action and/or the immune regulating action (or a composition comprising the same), which can produce the above superfine particles or composition. As a result, a pharmaceutical composition and a food and drink (food and/or drink) (health foods, functional foods etc.) etc. utilizing the active ingredient can be industrially and easily produced.

Accordingly, the present invention is extremely useful in industry, particularly in many fields such as medical practices (medical treatments), pharmaceutical products (preparations), foods etc.

**Claims**

1. An aqueous dispersion including water and a dispersant and superfine particles comprising β-glucan and having an average diameter of 10 $\mu$m or less dispersed in the water through the action of the dispersant.

2. An aqueous dispersion according to claim 1 wherein the particles have an average diameter of 1 $\mu$m or less.

3. An aqueous dispersion according to claim 1 or claim 2 wherein the particles have an average diameter of 0.01 to 1 $\mu$m.

4. An aqueous dispersion according to any one of the previous claims wherein the superfine particles are in the form of micelles.

5. An aqueous dispersion according to any one of the preceding claims wherein the dispersant is an edible emulsifier.

6. An aqueous dispersion according to claim 5 wherein the emulsifier is lecithin.

7. An aqueous dispersion according to any one of the preceding claims comprising 1-20,000 mg sugar and 1-20,000 mg dispersant in every 100g of dispersion.

8. A process for producing an aqueous dispersion according to any one of claims 1 to 7 comprising mixing a dispersant and an aqueous solution containing β-glucan, and subjecting the resulting mixture to a step of superfine pulverization.

9. A process according to claim 8 wherein the aqueous solution initially contains aggregates which include β-glucan and the aggregates are dispersed through the action of the dispersant during the step of superfine pulverization.

10. A process according to claim 8 or claim 9 wherein a high pressure emulsifier is used in the superfine pulverization step to mix the dispersant and the solution, thereby to produce superfine particles having an average particle diameter of 1 $\mu$m or less.

11. A process according to any one of claims 8 to 10 wherein the aqueous solution containing β-glucan is obtained by extracting an edible mushroom with water, and filtering the extract so obtained.

12. A process according to claim 11 further comprising concentrating and/or cooling the filtrate, thereby to obtain an aqueous solution containing aggregates.

13. A process according to claim 11 or claim 12 wherein the mushroom is selected from
    Lentinus edodes
    Pleurotus ostreatus
    Pholiota nameko
    Flammulina velutipes
    Tricholoma matsutake
    Lyophyllum shimeji
    Schizophyllum commune
    Crepidotus variabilis
    Lyophyllum ulmarium
    Grifola umbellata
    G. frondosa
    Coriolus versicolor
    Fomes fomentarius
    Volvavella volvacea
    Auricularia aurcula-judae
    Ganoderma lucidum
    G. applanatum
    Fomitopsis pinicola
    Dictyophora indusiata
    Sparassis crispa
    Agaricus blazei, and
    Peziza vesiculosa.

**14.** A process according to claim 9 or 12 wherein the aggregates have a particle diameter of at least 50 μm.

**15.** A process according to any one of claims 8 to 14 wherein the dispersant is mixed with the aqueous solution containing a β-glucan in an amount such that the ratio by weight of the dispersant to the total sugar contained in the aqueous solution is 100 at most.

**16.** A process according to any one of claims 8 to 15, further comprising drying the dispersion, thereby to obtain superfine particles in a dried state.

**17.** Superfine particles as obtainable by the process of claim 16.

**18.** A pharmaceutical composition for oral administration comprising the aqueous dispersion of any one of claims 1 to 7 or the superfine particles of claim 17 and, optionally, a pharmaceutically acceptable carrier or excipient (bulking filler).

**19.** A food or drink comprising an aqueous dispersion of any one of claims 1 to 7, or the superfine particles of claim 17.

**20.** A food or drink according to claim 19 which comprises the superfine particles in an amount of 0.01 to 80% by weight based on total sugar.

**21.** Use of an aqueous dispersion of any of claims 1 to 7, or of superfine particles of claim 17 in the manufacture of a pharmaceutical composition for oral administration, food or drink for activating or regulating immunity.

**22.** Use of an aqueous dispersion of any of claims 1 to 7 or of superfine particles of claim 17 in the manufacture of a pharmaceutical composition for oral administration, food or drink, for use in patients with a disease selected from a cancer disease, a microbial infectious disease, a viral infectious disease, an autoimmune disease, diabetes, an allergic disease, and digestive organ diseases (including irritable bowel syndrome (IBS), inflammatory bowel disease (IBD), constipation, and diarrhea).

**Patentansprüche**

**1.** Wässerige Dispersion, die Wasser und ein Dispergiermittel und durch die Wirkung des Dispergiermittels in dem Wasser dispergierte superfeine Teilchen, die β-Glucan enthalten und einen mittleren Durchmesser von 10 μm oder weniger haben, enthalten.

**2.** Wässerige Dispersion nach Anspruch 1, worin die Teilchen einen mittleren Durchmesser von 1 μm oder weniger haben.

**3.** Wässerige Dispersion nach Anspruch 1 oder 2, worin die Teilchen einen mittleren Durchmesser von 0,01 bis 1 μm haben.

**4.** Wässerige Dispersion nach einem der vorstehenden Ansprüche, worin die superfeinen Teilchen in der Form von Micellen sind.

**5.** Wässerige Dispersion nach einem der vorstehenden Ansprüche, worin das Dispergiermittel ein eßbares Emulgiermittel ist.

**6.** Wässerige Dispersion nach Anspruch 5, wobei das Emulgiermittel Lecithin ist.

**7.** Wässerige Dispersion nach einem der vorstehenden Ansprüche, die 1 bis 20.000 mg Zucker und 1 bis 20.000 mg Dispergiermittel pro 100 g der Dispersion enthält.

**8.** Verfahren zum Herstellen einer wässerigen Dispersion nach einem der Ansprüche 1 bis 7, welches das Mischen eines Dispergiermittels und einer β-Glucan enthaltenden wässerigen Lösung und das superfeine Pulverisieren des erhaltenen Gemisches umfaßt.

**9.** Verfahren nach Anspruch 8, wobei die wässerige Lösung ursprünglich Aggregate enthält, die β-Glucan enthalten, und die Aggregate durch die Wirkung des Dispergiermittels während der Stufe der superfeinen Pulverisierung dispergiert werden.

10. Verfahren nach Anspruch 8 oder 9, wobei ein Hochdruckemulgiermittel in der Stufe der superfeinen Pulverisierung eingesetzt wird, um das Dispergiermittel und die Lösung zu mischen, wodurch superfeine Teilchen mit einem mittleren Teilchendurchmesser von 1 μm oder weniger hergestellt werden.

11. Verfahren nach einem der Ansprüche 8 bis 10, wobei die β-Glucan enthaltende wässerige Lösung durch Extrahieren eines eßbaren Pilzes mit Wasser und Filtrieren des so erhaltenen Extrakts erhalten wird.

12. Verfahren nach Anspruch 11, welches außerdem das Konzentrieren und/oder Kühlen des Filtrats umfaßt, wodurch eine Aggregate enthaltende wässerige Lösung erhalten wird.

13. Verfahren nach Anspruch 11 oder 12, wobei der Pilz unter
Lentinus edodes
Pleurotus ostreatus
Pholiota nameko
Flammulina velutipes
Tricholoma matsutake
Lyophyllum shimeji
Schizophyllum commune
Crepidotus variabilis
Lyophyllum ulmarium
Grifola umbellata
G. frondosa
Coriolus versicolor
Fomes fomentarius
Volvavella volvacea
Auricularia aurcula-judae
Ganoderma lucidum
G. applanatum
Fomitopsis pinicola
Dictyophora indusiata
Sparassis crispa
Agaricus blazei und
Peziza vesiculosa
ausgewählt ist.

14. Verfahren nach Anspruch 9 oder 12, wobei die Aggregate einen Teilchendurchmesser von mindestens 50 μm haben.

15. Verfahren nach einem der Ansprüche 8 bis 14, wobei das Dispergiermittel mit der ein β-Glucan enthaltenden wässerigen Lösung in einer Menge gemischt wird, so daß das Gewichtsverhältnis des Dispergiermittels zu dem in der wässerigen Lösung enthaltenen Gesamtzucker höchstens 100 ist.

16. Verfahren nach einem der Ansprüche 8 bis 15, welches außerdem das Trocknen der Dispersion umfaßt, wodurch superfeine Teilchen in einem trockenen Zustand erhalten werden.

17. Durch das Verfahren nach Anspruch 16 erhältliche superfeine Teilchen.

18. Arzneimittelzusammensetzung für die orale Verabreichung, welche die wässerige Dispersion nach einem der Ansprüche 1 bis 7 oder die superfeinen Teilchen nach Anspruch 17 und gegebenenfalls einen pharmazeutisch geeigneten Träger oder Corrigens (Füllmittel) umfaßt.

19. Nahrungsmittel oder Getränk, das eine wässerige Dispersion nach einem der Ansprüche 1 bis 7 oder die superfeinen Teilchen nach Anspruch 17 enthält.

20. Nahrungsmittel oder Getränk nach Anspruch 19, welches die superfeinen Teilchen in einer Menge von 0,01 bis 80 Gew.-%, bezogen auf den Gesamtzucker, enthält.

**21.** Verwendung einer wässerigen Dispersion nach einem der Ansprüche 1 bis 7 oder von superfeinen Teilchen nach Anspruch 17 bei der Herstellung einer Arzneimittelzusammensetzung für die orale Verabreichung, eines Nahrungsmittels oder Getränks für die Aktivierung oder Regulierung der Immunität.

**22.** Verwendung einer wässerigen Dispersion nach einem der Ansprüche 1 bis 7 oder von superfeinen Teilchen nach Anspruch 17 bei der Herstellung einer Arzneimittelzusammensetzung für die orale Verabreichung, eines Nahrungsmittels oder Getränks für die Verwendung in Patienten mit einer Krankheit, die unter einer Krebserkrankung, einer durch Mikroorganismen verursachten Infektionserkrankung, einer von Viren verursachten Infektionserkrankung, einer Autoimmunkrankheit, Diabetes, einer allergischen Erkrankung und Verdauungsorgankrankheiten (einschließlich Reizdarmsyndrom (IBS), Darmentzündung (IBD), Verstopfung und Durchfall) ausgewählt ist.

## Revendications

**1.** Dispersion aqueuse comprenant de l'eau et un dispersant et des particules superfines comprenant du β-glucane et ayant un diamètre moyen de 10 μm ou moins dispersées dans l'eau par l'action du dispersant.

**2.** Dispersion aqueuse selon la revendication 1, dans laquelle les particules ont un diamètre moyen de 1 μm ou moins.

**3.** Dispersion aqueuse selon la revendication 1 ou la revendication 2, dans laquelle les particules ont un diamètre moyen de 0,01 à 1 μm.

**4.** Dispersion aqueuse selon l'une quelconque des revendications précédentes, dans laquelle les particules superfines sont sous la forme de micelles.

**5.** Dispersion aqueuse selon l'une quelconque des revendications précédentes, dans laquelle le dispersant est un émulsifiant comestible.

**6.** Dispersion aqueuse selon la revendication 5, dans laquelle l'émulsifiant est la lécithine.

**7.** Dispersion aqueuse selon l'une quelconque des revendications précédentes, comprenant 1 à 20 000 mg de sucre et 1 à 20 000 mg de dispersant tous les 100 g de dispersion.

**8.** Procédé de production d'une dispersion aqueuse selon l'une quelconque des revendications 1 à 7, comprenant le mélange d'un dispersant et d'une solution aqueuse contenant du β-glutane, et la soumission du mélange résultant à une étape de pulvérisation superfine.

**9.** Procédé selon la revendication 8, dans lequel la solution aqueuse contient initialement des agrégats qui comprennent du β-glucane et les agrégats sont dispersés par l'action du dispersant pendant l'étape de pulvérisation superfine.

**10.** Procédé selon la revendication 8 ou la revendication 9, dans lequel un émulsifiant haute pression est utilisé dans l'étape de pulvérisation superfine pour mélanger le dispersant et la solution, pour produire ainsi des particules superfines ayant un diamètre moyen de particule de 1 μm ou moins.

**11.** Procédé selon l'une quelconque des revendications 8 à 10, dans lequel la solution aqueuse contenant du β-glucane est obtenue en extrayant un champignon comestible avec de l'eau et en filtrant l'extrait ainsi obtenu.

**12.** Procédé selon la revendication 11, comprenant en outre la concentration et/ou le refroidissement du filtrat, pour obtenir ainsi une solution aqueuse contenant des agrégats.

**13.** Procédé selon la revendication 11 ou la revendication 12, dans lequel le champignon est choisi parmi
Lentinus edodes
Pleurotus ostreatus
Pholiota nameko
Flammulina velutipes
Tricholoma matsutake
Lyophyllum shimeji
Schizophyllum commune

Crepidotus variabilis
Lyophyllum ulmarium
Grifola Umbellata
G. frondosa
Goriolus versicolor
Fomes fomentarius
Volvavella volvacea
Auricularia aurcula-judae
Ganoderma lucidum
G. applanatum
Fomitopsis pinicola
Dictyophora indusiata
Sparassis crispa
Agaricus Biazei, et
Peziza vesiculosa.

14. Procédé selon la revendication 9 ou 12, dans lequel les agrégats ont un diamètre de particule d'au moins 50 μm.

15. Procédé selon l'une quelconque des revendications 8 à 14, dans lequel le dispersant est mélangé avec une solution aqueuse contenant un β-glucane en une quantité telle que le rapport en poids du dispersant sur le sucre total contenu dans la solution aqueuse est de 100 au plus.

16. Procédé selon l'une quelconque des revendications 8 à 15, comprenant en outre le séchage de la dispersion, pour obtenir ainsi des particules superfines à l'état séché.

17. Particules superfines telles qu'elles peuvent être obtenues par le procédé de la revendication 16.

18. Composition pharmaceutique pour une administration orale comprenant la dispersion aqueuse selon l'une quelconque des revendications 1 à 7 ou les particules superfines de la revendication 17 et, facultativement, un véhicule ou excipient pharmaceutiquement acceptable (charge de dilution).

19. Aliment ou boisson comprenant une dispersion aqueuse selon l'une quelconque des revendications 1 à 7, ou les particules superfines selon la revendication 17.

20. Aliment ou boisson selon la revendication 19, qui comprend les particules superfines en une quantité de 0,01 à 80 % en poids sur la base du sucre total.

21. Utilisation d'une dispersion aqueuse selon l'une quelconque des revendications 1 à 7, ou des particules superfines selon la revendication 17, dans la fabrication d'une composition pharmaceutique pour une administration orale, d'une boisson ou d'un aliment pour activer ou réguler l'immunité.

22. Utilisation d'une dispersion aqueuse selon l'une quelconque des revendications 1 à 7, ou des particules superfines selon la revendication 17 dans la fabrication d'une composition pharmaceutique pour une administration orale, d'un aliment ou d'une boisson, pour l'utiliser chez des patients atteints d'une maladie choisie parmi une maladie cancéreuse, une maladie infectieuse microbienne, une maladie infectieuse virale, une maladie autoimmunitaire, le diabète, une maladie allergique et des maladies des organes digestifs (comprenant le syndrome du côlon irritable (CCI), l'affection abdominale inflammatoire (AAI), la constipation et la diarrhée).

# FIG. 1 (a)

## SHIITAKE EXTRACT

# FIG. 1 (b)

## MICELLAR SHIITAKE EXTRACT

PARTICLE SIZE DISTRIBUTION OF SHIITAKE EXTRACT
AND MICELLAR SHIITAKE EXTRACT

# FIG. 2 (a)

β-GLUCAN SOLUTION

# FIG. 2 (b)

MICELLAR β-GLUCAN

PARTICLE SIZE DISTRIBUTION OF
β-GLUCAN SOLUTION AND MICELLAR β-GLUCAN

EP 1 389 466 B1